# EUROPÄISCHE PATENTSCHRIFT

(11) **EP 1 706 157 B1**
(45) Veröffentlichungstag und Bekanntmachung des Hinweises auf die Patenterteilung: **17.08.2016**
(21) Anmeldenummer: 04816270.5
(22) Anmeldetag: 13.12.2004
(51) Int. Cl.: A61L 27/38

(54) **VERFAHREN ZUR HERSTELLUNG VON BANDSCHEIBENZELLTRANSPLANTATEN UND DEREN ANWENDUNG ALS TRANSPLANTATIONSMATERIAL**
METHOD FOR THE PRODUCTION OF INTERVERTEBRAL DISK TISSUE FROM DEGENERATE INTERVERTEBRAL DISK TISSUE
PROCEDE DE FABRICATION DE TRANSPLANTS DE CELLULES DISCALES ET UTILISATION EN TANT QUE MATERIAU DE TRANSPLANTATION

(30) Priorität: 12.12.2003 DE 10359830; 17.02.2004 US 544315 P; 06.09.2004 DE 102004043449
(43) Veröffentlichungstag der Anmeldung: 04.10.2006
(73) Patentinhaber: co.don AG, 14513 Teltow (DE)
(72) Erfinder: JOSIMOVIC-ALASEVIC, Olivera, 14195 Berlin (DE); LIBERA, Jeanette, 13156 Berlin (DE); METHNER, Vilma, 14532 Kleinmachnow (DE); MEISEL, Hans-Jörg, 14163 Berlin (DE)
(74) Vertreter: Simandi, Claus
(86) Internationale Anmeldenummer: PCT/DE2004/002761
(87) Internationale Veröffentlichungsnummer: WO 2005/055877

(56) Entgegenhaltungen:
- WO-A-02/22185
- US-A- 6 080 579
- US-A1- 2003 153 078
- US-A1- 2003 165 473
- US-A1- 2003 229 400
- US-B1- 6 340 369
- REINECKE J A ET AL: "In vitro transfer of genes into spinal tissues" ZEITSCHRIFT FUER ORTHOPAEDIE UND IHRE GRENZGEBIETE, ENKE, STUTTGART, DE, Bd. 135, Nr. 5, 1997, Seiten 412-416, XP001064896 ISSN: 0044-3220
- OKUMA M ET AL: "REINSERTION OF STIMULATED NUCLEUS PULPOSUS CELLS RETARDS INTERVERTEBRAL DISC DEGENERATION: AN IN VITRO AND IN VIVO EXPERIMENTAL STUDY" JOURNAL OF ORTHOPAEDIC RESEARCH, THE JOURNAL OF BONE AND JOINT SURGERY, INC.,, US, Bd. 18, Nr. 18, 2000, Seiten 988-997, XP001064826 ISSN: 0736-0266

## Beschreibung

Die Erfindung betrifft ein Verfahren zur Herstellung von autologen Gemisch-Bandscheibenzelltransplantaten wie in den Ansprüchen beschrieben, sowie ein mit diesem Verfahren erhältliches autologes Gemisch-Bandscheibenzelltransplantat.

Die Erfindung betrifft ein Verfahren zur in vitro Herstellung von Bandscheibenknorpelzelltransplantaten aus erkranktem Bandscheibengewebe von Patienten und dessen Anwendung als Transplantationsmaterial zur Behandlung von erkrankten Bandscheiben. Die Erfindung betrifft weiterhin dreidimensionales, vitales und mechanisch stabiles Bandscheibenknorpelgewebe und dessen Verwendung als Transplantationsmaterial zur Behandlung von erkrankten Bandscheiben sowie zur Testung von Wirkstoffen. Gegenstand der Erfindung sind weiterhin die hergestellten Bandscheibenzelltransplantate und die Transplantationstechnik und die hergestellten Bandscheibenknorpelgewebe und therapeutische Zubereitungen, z.B. Injektionslösungen, die dieses Gewebe und die Zelltransplantate beinhalten.

Die Degeneration der Bandscheiben wird während der Alterung oder durch Trauma ausgelöst und induziert akute und chronische Schmerzen und Instabilitäten in der Wirbelsäule. Mehr als 300.000 Patienten in Europa leiden an Bandscheibenerkrankungen. Ungefähr 70 % der Patienten, die einen Bandscheibenvorfall erleiden und mittels Discectomie behahndelt werden, leiden weiterhin an Rückenschmerzen. Anhaltende starke Schmerzen machen bei 10 % dieser Patienten eine weitere operative Behandlung notwendig (Yorimitsu et al, 2001). Grund dafür ist die durch die Operation bedingte Abnahme der Bandscheibenhöhe, die damit verbundene Erhöhung der lokalen Belastund des Bandscheibengewebes (Brinckmann und Grootenboer, 1991) und vor allem die fehlende Heilung und Regeneration des zerstörten und entfernten Bandscheibengewebes (Lundon und Bolton, 2001, Meakin et al., 2001). Mit fortschreitender Zeit resultiert diese Instabilität der betroffenen Bandscheibe in degenerativen Veränderungen angrenzender Bandscheiben, wodurch weitere operative Eingriffe und im schlimmsten Fall eine Fusion der Wirbelkörper oder das Einsetzen einer Prothese notwendig werden. Deshalb ist die biologische Reparatur bzw. Regeneration der Bandscheibe die Zukunft für die Behandlung degenerierter Bandscheiben.

Eine bekannte Methode zur biologischen Regeneration eines Gewebes ist die Knorpelzelltransplantation unter Verwendung von körpereigenen Zellen, die zur Behandlung von Gelenksknorpeldefekten angewandt wird. Dabei wird das Potential der Gelenksknorpelzellen genutzt, um *in vivo* nach Transplantation der Zellen neues Gewebe aufzubauen. Dazu wird dem Patienten eine Gelenksknorpelbiopsie entnommen, daraus Knorpelzellen isoliert, mittels Zellkultivierung vermehrt und anschließend dem Patienten im Bereich des Gewebedefektes, z.B. durch Einspritzen, transplantiert. Dort bilden sie neues Gewebe und füllen somit den Defekt vollständig auf. Durch diese Verfahren wird erreicht, dass nach Applikation eines Zelltransplantates im Körper Gewebe aufgebaut wird. Prinzipiell ist diese Methodik für die Behandlung der Bandscheibendegeneration nicht anwendbar, da den Patienten aus ethischen Gründen kein Ausgangsmaterial aus einer intakten benachbarten Bandscheibe entnommen werden kann und krankes Gewebe a priori nicht verwendbar ist.

Erste Ansätze zum biologischen Ersatz der Bandscheibe gehen von der Verwendung von gesundem Bandscheibengewebe aus. So beschreiben Handley (US 6,080,579) und Ferree (US 6,340,369 B1) die Verwendung von normalem Bandscheibengewebe für die Isolation von Bandscheibenzellen und die Kombination dieser Zellen mit einem bioresorbierbaren Träger. Auch viele wissenschaftliche Arbeiten haben die Verwendung von normalem Bandscheibengewebe als Grundlage: Okuma et al., 2000, Gruber et al., 2000, Chelberg et al., 1995. Jedoch kann eine gesunde Bandscheibe eines Patienten nicht als Gewebequelle zur Behandlung einer anderen Bandscheibe dienen, da die Gewebeentfernung zu einer Zerstörung, zur Degeneration und somit zum Verlust der Funktion dieser Bandscheibe führt.
Ein anderer Ansatz ist die Verwendung von degeneriertem Nukleus Pulposus Gewebe, welches aus dem Inneren einer degenerierten Bandscheibe entfernt und aufgearbeitet wird. Bei diesem operativen Eingriff wird die ohnehin schon degenerierte und zerstörte Bandscheibe noch weiter zerstört. Die Aufarbeitung des Gewebes wird dabei zum einen vorgeschlagen als Dehydrierung (US 6,648,918) oder die Kombination von darin befindlichen Zellen mit einem Trägermaterial (US 6,569,442; US2001/0020476 A1) und die anschliessende Transplantation zurück in diese degenerierten Bandscheiben. Allerdings enthält das dehydrierte Gewebe keine lebenden Zellen und stellt somit keine Methode der biologischen Regeneration dar. Die vorgeschlagene Kombination der Nukleus Zellen oder anderer Zellen mit einem Trägermaterial stellt ebenfalls keine reine biologische Methode dar und bedingt die Verwendung eines geeigneten Trägermaterials, welches zum Beispiel biomechanisch geeignet sein muss, welches im selben Masse wie neues Gewebe aufgebaut werden soll abgebaut wird, welches die Bildung neuen Gewebes nicht behindern darf oder zum Beispiel keine immunologische Reaktion aufgrund der auf jeden Fall verwendeten synthetischen, allogenen oder xenogenen Materialien auslösen darf.
Eine weitere Behandlungsmöglichkeit wäre die Verwendung von Bandscheiben anderer Patienten, wobei es sich somit um eine allogene Transplantation handelt (6,344,058; Keith DK et al., 2003). Hierbei stellt jedoch die immunologische Reaktion ein Problem dar und durch die alleinige Einbringung einer Spenderbandscheibe wird wahrscheinlich auch keine biologische Regeneration der betroffenen Bandscheibe ausgelöst.

US2003165473 beschreibt die Herstellung von Bandscheibengewebe aus Zellen des Anulus Fibrosus, des Nucleus Pulposus und der Endplatte.

Aus den genannten Problemen bestehen deshalb für die Behandlung von degenerierten Bandscheiben und zur Bandscheiberegeneration die allgemeinen Ziele darin: medizinisch und ethisch vertretbares Ausgangsgewebe bzw. - zellprobe zu verwenden, keine anderen oder die betroffene, erkrankte Bandscheibe des Patienten zu zerstören, nur patienteneigene Materialien zu verwenden (autologe Therapie), optimale Zellisolationsbedingungen und - kultivierungsbedingungen zur Vermehrung der Bandscheibenzekllen und anschliessender Bandscheibenmatrixbildung zu finden, zur Vermeidung von Immunreaktionen auf Trägermaterialien zu verzichten. Diese Probleme sind lösbar durch die Transplantation eines speziellen autologen Zelltransplantates oder durch Transplantation eines außerhalb des Körpers vorgefertigten 3-dimensionalen Bandscheibenknorpelgewebes.

Aufgabe der vorliegenden Erfindung war es deshalb, Verfahren zur Herstellung von Bandscheibenknorpelzelltransplantaten und stabilem vitalen Bandscheibenknorpelgewebe bereitzustellen, die zur autologen Transplantation, zum schnellen Wiederaufbau und der Erhaltung der Funktion der Bandscheibe geeignet sind. Dabei ist es essentiell, dass das Ausgangsmaterial zur Herstellung der Zelltransplantate medizinisch und ethisch vertretbar entnommen werden kann sowie, dass die autolog kultivierten Bandscheibenzellen ihre Eigenschaften über den Zeitraum von der Entnahme bis zur Transplantation nicht verändern und eine hohe Proliferations- und Differenzierungskapazität aufweisen.

Überraschend konnte gezeigt werden, dass als Ausgangsmaterial erkranktes Bandscheibengewebe verwendet werden kann. Bisher wurde angenommen, dass es nicht möglich ist, aus degeneriertem Gewebe, adulte Zellen in ausreichender Anzahl zu isolieren, die vital sind, ausreichend proliferieren und anschließend auch noch in der Lage sind, gewebespezifisch zu differenzieren und Bandscheibengewebe aufzubauen, da in Geweben, die einer Degeneration unterliegen die gewebespezifische Zellen ihre Eigenschaften im Hinblick auf die Matrixsynthese verändern und auch absterben und auch durch andere Zellen mit anderen gewebe-unspezifischen Eigenschaften ersetzt werden. Überraschenderweise konnte jedoch insbesondere aus vorgefallenem degenerierten Bandscheibengewebe eine ausreichende Anzahl an vitalen Zellen isoliert werden. Das vorgefallene degenerierte Bandscheibengewebe besteht aus Bandscheibenteilen des Anulus Fibrosus und Nucleus Pulposus, wobei die aus beiden Gewebebereichen isolierten Zellen (Anulus Fibrosus Zellen und Nucleus Pulposus Zellen) unter den gegebenen autologen Kulturbedingungen dann auch noch proliferieren und gewebespezifisch besonders spezifisch differenzieren. Damit sind diese Gemischzelltransplantate für eine zell-basierte Therapie zur Wiederherstellung der Funktion der Bandscheibe geeignet.

Es ist somit erstmals ein Verfahren beschrieben, durch welches besondere autologe Gemisch-Bandscheibenzelltransplantate hergestellt werden können, die nach Transplantation in eine geschädigte/ erkrankte Bandscheibe durch den Aufbau neuen Bandscheibengewebes die Erhaltung der Bandscheibe und damit die Wiederherstellung der neurologischen und mechanischen Funktion der Wirbelsäule bei einer Bandscheibenerkrankung oder nach einem Bandscheibenvorfall erlaubt.

Auch bei fortgeschrittener Degeneration der Bandscheibe, d.h. auch bei Degeneration oder traumatischer Schädigung der äußeren Schicht der Bandscheibe (Anulus Fibrosus), ist durch die vorliegende Erfindung die Wiederherstellung und Erhaltung der neurologischen, biologischen und mechanischen Funktion der Bandscheibe ermöglicht, wenn aus vorgefallenen, degenerierten Bandscheiben Gemisch-Gewebezellen isoliert werden, die folgend zu einem autologen 3-dimensionalen Gewebe ohne die Verwendung von Trägermatieralien kultiviert werden. Das isolierte Bandscheibengewebe, d.h. die Bandscheibenzellen, werden unter autologen Kultivierungsbedingungen nur unter Zusatz von patienteneigenem Serum im Zellkulturmedium in Monolayer vermehrt. Bevorzugt sind die aus dem degenerierten, vorgefallenen Bandscheibengewebe isolierten Bandscheibenzellen während der Vermehrung in einem Zellkulturmedium, das 1-20% autologen Serumzusatz enthält und dessen Verhältnis von aplha-MEM-Medium und HAM-F12-Medium zwischen 2:1 und 1:2 beträgt, und bei 36.8-37°C, 5% Kohlendioxid Luftanteil und einer Luftfeuchte von 85-95% kultiviert, wodurch sich ihre Synthese an Matrix- und Markerproteinen nicht ändert.

Bevorzugt ist weiterhin, dass die isolierten Bandscheibenzellen nach ihrer Vermehrung in Monolayer in einem Zellkulturmedium, das 1-20% autologen Serumzusatz enthält und dessen Verhältnis von aplha-MEM-Medium und HAM-F12-Medium zwischen 2:1 und 1:2 beträgt, und bei 36.8-37°C, 5% Kohlendioxid Luftanteil und einer Luftfeuchte von 85-95% kultiviert und dadurch differenzierungsfähig sind und Matrixstrukturen bilden, die spezifische Bandscheibenmatrixproteine umfassen.

Bevorzugt ist weiterhin, dass die isolierten Bandscheibenzellen nach deren Vermehrung in Monolayer in einer Lösung aus 10 % DMSO, 20 % Serum und 70 % Kulturmedium eingefroren und wieder aufgetaut werden und so deren Eigenschaften im Hinblick auf die Synthese von spezifischen Matrixkomponenten und Markern nicht verändern und Gewebestrukturen in vitro und in vivo aufbauen, die aus Bandscheiben-spezifischen Matrixproteinen bestehen.

Die beschriebenen Merkmale der nicht veränderten Synthese von Marker- und Matrixproteinen stellen keine Aufgabe oder gewünschte Funktion dar, die erzielt werden soll, sondern die Folge der Kultivierungsschritte. Die Nennung dieser Merkmale erfolgt lediglich aus Gründen der Klarstellung. Mit der Offenbarung dieser Merkmale soll demgemäß klargestellt werden, welche Folgen die erfindungsgemäßen Verwendungs- oder Verfahrensschritte haben.

Die Bildung der Matrixstrukturen, die spezifische Bandscheibenmatrixproteine umfassen, ist daher keine erstrebenswerte Eigenschaft, sondern die Bildung der genannten Matrixsturkturen ergibt sich aus den Kultivierungsbedingungen.

Bevorzugt werden die die aus dem Bandscheibengewebe isolierten Zellen in einem Kulturgefäß mit hydrophober Oberfläche und sich verjüngendem Boden kultiviert, wodurch die dreidimensionalen Zellaggregate erhalten werden.

Bei der Behandlung mit den erfindungsgemäßen Bandscheibenzelltransplantaten ist es vorteilhaft, wenn vor der Transplantation die äußere Hülle der Bandscheibe, der Anulus Fibrosus, der durch den Austritt des Bandscheibengewebes geschädigt wurde, in der Art heilt, dass keine Flüssigkeit, wie die hergestellten Zelltransplantate, aus dem Inneren der Bandscheibe mehr auslaufen kann. Dieser Zeitraum ist patientenabhängig. Während dieses Zeitraumes werden die Bandscheibenzelltransplantate hergestellt, wobei die Gemisch-Bandscheibenzellen während der Vermehung in der Zellkultur ihre gewebe-spezifischen Eigenschaften im Hinblick auf deren Differenzierungspotential und damit den Erfolg der Transplantation erhalten. Im Gegensatz dazu wird dieses Potential bei der getrennten Kultivierung der Anulus Fibrosus und Nucleus Pulposus Zellen verringert, wobei nach Überführung in die 3-dimensionale Umgebung einige Bandscheiben-spezifische Marker nicht exprimiert werden. Deshalb sind nur die Gemisch-Kulturen besonders geeignet Bandscheibengewebe nach deren Transplantation in eine degenerierte Bandscheibe aufzubauen.

Außerdem sollen die in vitro hergestellten Zell- und Gewebetransplantate keine immunologischen Reaktionen im Organismus, der das Transplantat erhält, auslösen. Es wurde überraschender Weise gefunden, dass diese erfindungsgemäß autolog hergestellten Zellen und Gewebe keine immunologische Reaktionen auslösen.

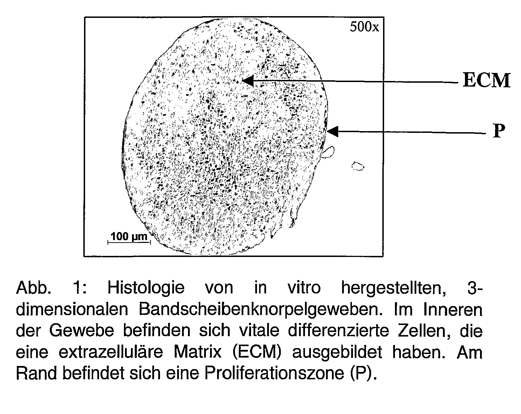

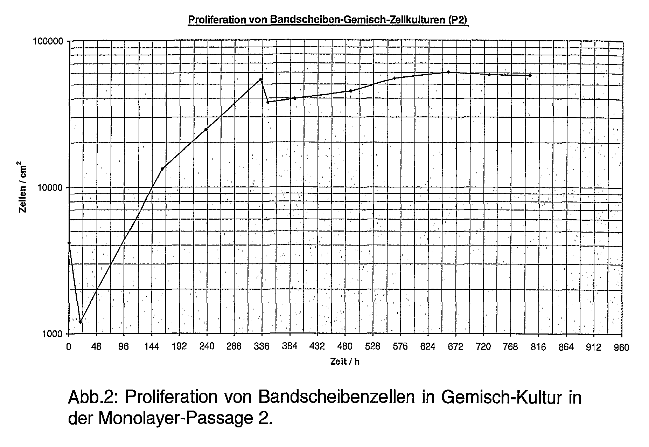

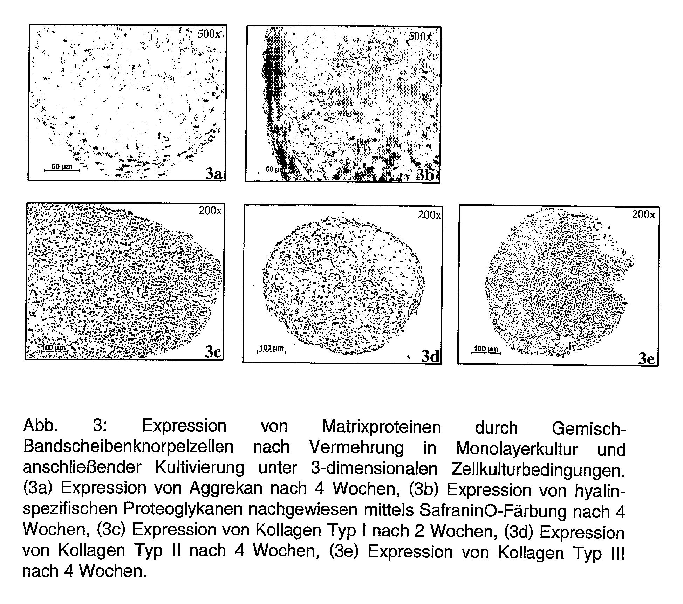

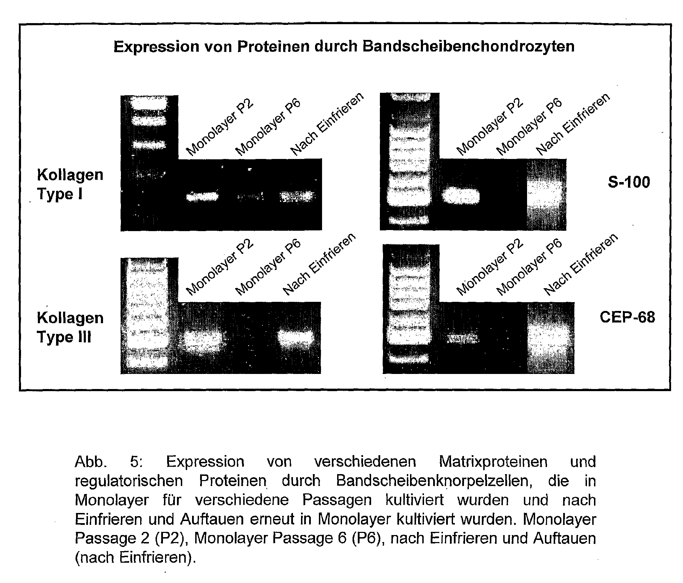

Das entnommene, patienteneigene erkrankte Bandscheibengewebe kann unterschiedlich weiter bearbeitet werden:
(a) Aus den Biopsien werden die Gemisch-Bandscheibenzellen mittels enzymatischem Verdau des Gewebes, durch Auswandern oder durch Reagenzien, die Zielzellen erkennen, mit üblichen Methoden isoliert. Diese Zellen werden dann nur unter Zusatz von körpereigenem Serum und ohne Zusatz von exogenen wachstumsfördernden Verbindungen und ohne den Zusatz von Antibiotika und mit üblichem Kulturmedium in Zellkulturgefäßen kultiviert und so lange vermehrt, bis eine ausreichende Menge an Zellen zur Verfügung steht (Abb. 2). Diese Zeit wird so kurz wie möglich gehalten, um ihre phänotypischen Eigenschaften nicht zu verändern. Nach ausreichender Vermehrung der Zellen werden diese geerntet und das Zelltransplantat bestehend aus einer Bandscheibenzell-suspension ist für therapeutische Verwendungen bereitgestellt.
   Erfindungsgemäß werden die isolierten Bandscheibenzellen im Gemisch verwendet, d.h. die Zellen werden nicht nach Anulus Fibrosus und Nucleus Pulposus Zellen, deren Gewebeanteile im vorgefallenen Bandscheibengewebe enthalten sind, aufgetrennt und getrennt oder nur als Einzelzellart kultiviert. Genau unter diesen Gemisch-Bedingungen wird eine spätere verbesserte Bandscheiben-spezifische Differenzierung der Zellen erreicht (siehe Abb. 3). Diese autologe Gemisch-Kultiverungstechnik zur Vermehrung der Bandscheibenzellen ermöglicht somit erstmals eine Bandscheiben-spezifische Differenzierung dieser so kultivierten Zellen, nachdem diese in eine 3-dimensionale Umgebung überführt werden.
(b) In einem weiteren Verfahren werden die isolierten Bandscheibenzellen vorkultiviert und ohne Passagierung der Zellen nur kurzzeitig vermehrt. Danach werden die vorkultivierten Zellen geerntet und eingefroren und bis zum Zeitpunkt der Transplantation tiefgefroren gelagert. Vor der Transplantation werden die Zellen aufgetaut und bis zum Erreichen einer ausreichenden Zellzahl mit autologem Serum und in herkömmlichem Zellkulturmedium weiterkultiviert. Nach ausreichender Vermehrung der Zellen werden diese geerntet und das Zelltransplantat bestehend aus einer Bandscheibenzellsuspension bereitgestellt. Überraschenderweise wurde festgestellt, dass die Bandscheibenzellen durch das Einfrieren und Auftauen nicht ihre spezifischen Eigenschaften im Hinblick auf die Synthese von spezifischen Marker- und Matrixproteinen verlieren (siehe Abb. 5).
(c) In einem weiteren bevorzugten Verfahren wird als Ausgangsmaterial ebenfalls patienteneigenes erkranktes Bandscheibengewebe verwendet. Aus den Biopsien werden die gewebeaufbauenden Zellen aus dem Anulus Fibrosus und Nucleus Pulposus mittels enzymatischem Verdau des Gewebes, durch Auswandern oder durch Reagenzien, die Zielzellen erkennen, mit üblichen Methoden isoliert. Diese Gemisch-Zellen werden dann unter autologen Bedingungen und mit üblichem Kulturmedium zunächst in Monolayer kultiviert bis eine ausreichende Zellzahl erreicht ist und anschließend in Zellkulturgefäße mit hydrophober Oberfläche und sich verjüngendem Boden überführt und dort so lange in Suspension kultiviert, bis ein dreidimensionales Zellaggregat entsteht, das zu mindestens 40 Volumen%, vorzugsweise mindestens 60 Volumen% bis maximal 99 Volumen%, extrazelluläre, de novo synthetisierte Matrix (ECM) beinhaltet, in welche differenzierte Zellen eingebettet sind. Der Fachmann kann diese Werte durch Entnahme kleinerer Proben bestimmen. Das entstandene Zellaggregat weist einen äußeren Bereich auf, in welchem proliferations- und migrationsfähige Zellen vorhanden sind (siehe Abb. 1).

Erfindungsgemäß werden die isolierten Bandscheibenzellen im Gemisch verwendet, d.h. die Zellen werden nicht nach Anulus Fibrosus und Nucleus Pulposus Zellen aufgetrennt und getrennt oder nur als Einzelzellart kultiviert. Genau unter diesen Gemisch-Bedingungen wird in der 3-dimensionalen Kultur und damit bei der Bildung der 3-dimensionalen Bandscheibengewebe die Banscheiben-spezifische Differenzierung der Gemischzellen gefördert, wobei die Expression von Bandscheiben-spezifischen Markern nur in diesen Kulturen erreicht werden kann und die Bildung der 3-dimensionalen Bandscheibengewebe bei Verwendung der Gemischkulturen gefördert ist (siehe Abb. 3). Diese autologe Gemisch-Kultiverungstechnik ermöglicht somit erstmals die Herstellung und Verwendung eines Bandscheibenspezifischen Gewebetransplantates, welches autolog aus degenerierem, vorgefallenen Bandscheibengewebe hergestellt wurde.

Die Gemisch-Bandscheibenzellen, die aus erkranktem Bandscheibengewebe isoliert werden und aus denen autologe 3-dimensionale Bandscheibenzellaggregate hergestellt werden, so dass die entnommenen Zellen in einem de novo synthetisierten Gewebe integriert sind, überleben auch mit fortschreitender Kultivierungsdauer, d. h. die Zellen im Inneren der Aggregate sterben nicht ab. Mit fortschreitender Kultivierungsdauer differenzieren die Zellen im Inneren der Aggregate aus und es bilden sich Bandscheibenknorpelgewebe, die aus ECM, differenzierten Zellen und einer Proliferationszone am Rand bestehen (siehe Abb. 1). Während der Differenzierung in der autologen Zellkultur wird der Abstand der aggregierten Zellen durch Bildung der gewebespezifischen Matrix immer größer (siehe Abb. 3 Vergleich (3c) mit (3d)). Es entsteht im Inneren der in vitro hergestellten Bandscheibengewebe eine Gewebehistologie, die dem natürlichen Gewebe sehr ähnlich ist. Während der weiteren Herstellung der Bandscheibenknorpelgewebe bildet sich die "Proliferationszone" am Rand selbiger aus. Diese Zone hat den unschätzbaren Vorteil, dass nach Einbringen der so entstandenen Bandscheibenknorpelgewebe in degenerierte Bandscheiben, die in dieser Randzone befindlichen Zellen in der Lage sind, auszuwandern und aktiv den Kontakt zum umliegenden Gewebe herzustellen bzw. eine Integration des in vitro gebildeten Bandscheibenknorpelgewebes in seine Umgebung ermöglichen. Damit sind die hergestellten gewebespezifischen Zellaggregate hervorragend zur Behandlung von degenerierten Bandscheiben und zum Neuaufbau von Bandscheibengewebes *in vivo* geeignet.

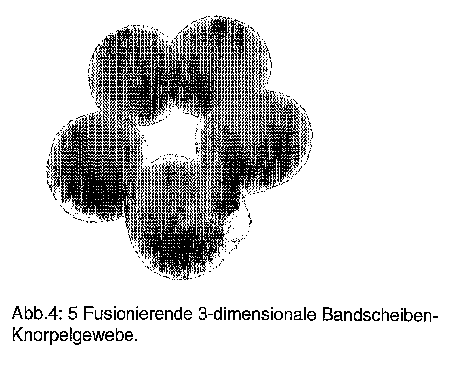

Aufgrund der biomechanischen Belastung der Bandscheiben direkt nach Behandlung sowie dem Ziel die Bandscheibe in ihrer Höhe gleich mit der Transplantation des Bandscheibenknorpelgewebes wiederherzustellen, kann es von Vorteil sein, bereits größere und mechanisch stabile Gewebestücke zu transplantieren. Für diesen Fall werden mindestens zwei, besser aber mehr der erhaltenen in vitro Bandscheibenknorpelgewebe fusioniert, indem sie gemeinsam unter den gleichen Bedingungen und in den gleichen Kulturgefäßen wie oben beschrieben bis zur gewünschten Größe weiterkultiviert werden (siehe auch Abb. 4).

Als Zellkulturmedium kann sowohl für die Suspensions- als auch für die Monolayerkultur übliches Medium, z. B. Dulbecco's MEM, unter Zusatz von Serum, verwendet werden. Vorzugsweise wird DMEM und Hams im Verhältnis 1:1 eingesetzt. Um jedoch immunologische Reaktionen des Patienten auf das in vitro hergestellte Gewebe zu vermeiden, wird als Serum autologes Serum des Patienten eingesetzt.

Dem Kulturmedium werden erfindungsgemäß keine Antibiotika, Fungistatika oder andere Hilfsstoffe zugesetzt.Es hat sich gezeigt, dass nur die autologe Kultivierung der Zellen und Zellaggregate sowie die Kultivierung ohne Antibiotika und Fungistatika eine unbeeinflußte Proliferation sowie Differenzierung der Zellen in der Monolayerkultur und eine ungestörte Bildung der spezifischen Matrix in den Zellaggregaten ermöglicht. Weiterhin sind durch den Verzicht von Zusatzstoffen während der Herstellung nach Einbringen des in vitro hergestellten Gewebes in den menschlichen und auch tierischen Organismus immunologische Reaktionen ausgeschlossen.

Die Größe der hergestellten Bandscheibengewebe hängt von der eingebrachten Zellzahl pro Volumen Kulturmedium ab. Werden beispielsweise 1 x 10⁷ Zellen in 300 µl Kulturmedium eingebracht, so entstehen innerhalb von 1 Woche dreidimensionale Bandscheibenzellaggregate von ca. 500-700 µm Durchmesser. Die andere Möglichkeit ist die in vitro-Fusion der kleinen Zellaggregate zu größeren - wie oben beschrieben - und das Einbringen dieser in die Bandscheibe. Vorzugsweise werden erfindungsgemäß zwischen 1 x 10⁴ und 1 x 10⁷ Zellen in 300 µl Kulturmedium zur Herstellung der kleinen Zellaggregate verwendet, besonders bevorzugt 1 x 10⁵ Zellen. Die nach einigen Tagen gebildeten in vitro Bandscheibengewebe werden dann für mindestens 2-4 Wochen in Abhängigkeit von der Zellart und den patientenspezifischen Charakteristika in dem geeigneten Kulturmedium kultiviert, um die Ausbildung der gewebespezifischen Matrix zu induzieren. Im besonderen Falle können dann einzelne in vitro Bandscheibengewebe ab ca. einer Woche Kultivierung fusioniert werden, um die Größe des Gewebestückes zu erhöhen.

Als Zellkulturgefäße kommen für die erfindungsgemäße Kultivierung in Suspension bevorzugt solche mit hydrophober, also adhäsionsverhindernder Oberfläche, in Betracht, wie z. B. Polystyrol oder Teflon. Zellkulturgefäße mit nichthydrophober Oberfläche können durch Beschichten mit Agar oder Agarose hydrophobiert werden. Weitere Zusätze sind nicht erforderlich. Vorzugsweise dienen als Zellkulturgefäße Napfplatten. Dabei können für die Herstellung der kleinen Zellaggregate beispielsweise 96-Napfplatten und für die Herstellung der fusionierten Aggregate 24-Napfplatten Verwendung finden.

Beschrieben ist auch die operative Technik zur Transplantation der Bandscheibenzellen und der in vitro hergestellten 3-dimensionalen Bandscheibenknorpelgewebe in die geschädigte Bandscheibe. Die Transplantation wird durch Injektion der Bandscheibenzellen unter fluoroskopischer Kontrolle nach Desinfektion der Haut, steriler Abdeckung des Hautareals und unter Lokalanästhesie und bevorzugt unbedingt unter Vermeiduung der Verwendung von Kontrastmitteln durchgeführt. Dazu werden die Bandscheibenzelltransplantate insbesondere nach deren Herstellung im Labor in speziellen Transportröhrchen mit sich verjüngendem Boden, abgerundet oder spitz, eingefüllt und im Operationsraum mittels einer Punktionsnadel mit z.B. schrägem Kanülenausgang in eine Spritze aufgezogen. Vor allem durch den schrägen Boden des Transportgefäßes und den schrägen Kanülenausgang ist eine vollständige Aufnahme der Zellen enthaltenden Lösung möglich. Zur Sicherstellung der Abgabe der Zellen in die Bandscheibe ohne Schädigung der Zellen und mit kleinstmöglichem Flüssigkeits- und somit Zellverlust hat die Punktionsnadel im Wesentlichen einen Innendurchmesser von 0,4 bis 2 mm. Die Transplantation durch Injektion der Bandscheibenzellen in die zu behandelnde Bandscheibe erfolgt im Speziellen auf der gegenüberliegenden Seite der zuvor operierten Seite der Bandscheibe (Bandscheibenvorfallentfernung) mittels einer Punktionsnadel mit schrägem Ausgang. Die Injektion des Zelltransplantates findet unter fluoroskopischer Kontrolle statt, da die tatsächliche Abgabe der Zellen in den Bandscheibeninnenraum kontrolliert werden muss. Herkömmlicherweise können Kontrastmittel verwendet werden, die jedoch die Zellen schädigen, wodurch der Erfolg der Zelltransplantation verhindert wird. Nach Abgabe der Bandscheibenzellen in den Bandscheibenraum wird die Punktionsnadel aus der Bandscheibe heraus gezogen. Für den Patienten erfolgt bevorzugt eine 12-stündige strikte Bettruhe, eine folgende 12-24-stündige reguläre Bettruhe und eine 24-48-stündige Bettruhe mit physiotherapeutischen Übungen. Anschließend erfolgt z.B. für einige Wochen die Stabilisierung der Wirbelsäule über eine herkömmliche geeignete Orthese.

Die Transplantation der 3-dimensionalen, in vitro hergestellten Bandscheibenknorpeltransplantate erfolgt wie für die Bandscheibenzellen insbesondere mittels einer Punktionsnadel. Zur Sicherstellung der Vermeidung einer mechanischen und damit biologischen Schädigung der Bandscheibenknorpeltransplantate während der Injektion, wird eine Punktionsnadel mit einem Durchmesser von mindestens 500µm verwendet. Weiterhin erfolgt zur Vermeidung der mechanischen Schädigung der Bandscheibenknorpeltransplantate im Wesentlichen nur ein einmaliges Passieren durch die Punktionsnadel. Deshalb werden die Bandscheibenknorpeltransplantate nach deren Herstellung im Labor bereits in eine Spritze überführt, auf die dann im Operationssaal nur noch die Punktionsnadel aufgesetzt wird. Auch hier muss die Punktionsnadel erfindungsgemäß einen schrägen Auslauf aufweisen, um durch die vergrößerte Ausgabefläche ein schnelles Abgeben der Bandscheibenknorpeltransplantate im kleinstmöglichen Flüssigkeitsvolumen (Abgabevolumen) zu ermöglichen. Anschließend erfolgt die Injektion wie oben für die Zelltransplantate beschrieben.

Gegenstand der Erfindung sind auch therapeutische Zubereitungen, die die erfindungsgemäßen Bandscheibenzelltransplantate und das Bandscheibenknorpelgewebe umfassen, z.B. Injektionslösungen.

Gegenstand der Erfindung ist auch die Verwendung des erfindungsgemäßen Bandscheibenknorpelgewebe zur Testung von Wirkstoffen, die z. B. die Bildung und Differenzierung von Matrix und Zellen beeinflussen. Dazu werden die Bandscheibenzellaggregate erfindungsgemäß hergestellt und in unterschiedlichen Reifestadien werden die zu testenden Medikamente hinzugegeben und unterschiedlichste Parameter der in vitro Bandscheibengewebeherstellung und -reifung charakterisiert. Diese Tests sind im Vergleich zu den herkömmlichen Medikamententests an Tieren oder Tumorzellsystemen durch die Verwendung von nur autologem Material patientenspezifisch und ermöglichen eine individuelle Diagnose.

Nachfolgend soll die Erfindung an Ausführungsbeispielen näher erklärt werden, ohne sie darauf einzuschränken.

### Ausführungsbeispiele

### Beispiel 1: Herstellung von Bandscheibenzelltransplantaten

Aus dem erkrankten Bandscheibengewebe werden mittels enzymatischen Verdaus durch Inkubation mit Kollagenaselösung die Bandscheibenknorpelzellen aus dem Anulus Fibrosus und Nucleus Pulposus isoliert. Nach Trennung der isolierten Zellen vom unverdauten Gewebe, werden diese als Gemischzellpopulationen in Zellkulturflaschen überführt und unter Zugabe von DMEM / Hams F12 Kulturmedium (1/1) und 10% autologem Serum des Patienten bei 37°C und 5% CO₂ inkubiert. Zweimal wöchentlich wird ein Mediumwechsel durchgeführt. Nach Erreichen des konfluenten Stadiums wird die Zellayer mit physiologischer Kochsalzlösung gewaschen und mittels Trypsin von der Zellkulturoberfläche geerntet. Nach einer weiteren Waschung werden die Bandscheibenzellen in physiologische Kochsalzlösung überführt und zur Transplantation zur Verfügung gestellt.

In einem in vitro Model konnte das Differenzierungspotential der im Zelltransplantat enthaltenen Bandscheibenzellen aufgezeigt werden. Es werden bandscheibenspezifische Matrixproteine und Markerproteine exprimiert (Abb. 3) und damit eine bandscheibenspezifische Gewebestruktur aufgebaut.

### Beispiel 2: Transplantation von Bandscheibenknorpelzellen

Die in Beispiel 1 hergestellten Bandscheibenzelltransplantate (mind. 1.000 Zellen, max. 100 Millionen Zellen) vorzugsweise ca. 1 Million Bandscheibenknorpelzellen wurden in physiologischer Kochsalzlösung aufgenommen und in die erkrankte Bandscheibe eingespritzt. Es wurde unter anderem festgestellt, dass in der behandelten Bandscheibe der Wassergehalt wieder ansteigt, sowie die Höhe des Zwischenwirbelraumes aufrechterhalten werden kann, was beides auf die durch die Bandscheibenknorpelzellen synthetisierten Matrixproteine zurückzuführen ist.

Die erfindungsgemäße in vitro hergestellte Bandscheibenzelltransplantate werden von den Patienten angenommen, gewährleisten eine schnelle Integration der proliferationsfähigen und migrationsfähigen Zellen sowie eine Regeneration des Bandscheibengewebes durch die Differenzierungsfähigkeit der enthaltenen Zellen. Somit erlauben die Bandscheibenzelltransplantate den schnellen Wiederaufbau von Bandscheibengewebe, eine schnelle Genesung der Patienten und die Wiederherstellung der Bandscheibenfunktion.

### Beispiel 3: in vitro Herstellung von Bandscheibenknorpelgewebe

Aus dem vorgefallenen Bandscheibengewebe werden mittels enzymatischen Verdaus durch Inkubation mit Kollagenaselösung die Bandscheibenknorpelzellen aus dem Anulus Fibrosus und Nucleus Pulposus isoliert. Nach Trennung der isolierten Zellen vom unverdauten Gewebe, werden diese als Gemischkultur in Zellkulturflaschen überführt und unter Zugabe von DMEM / Hams F12 Kulturmedium (1/1) und 10% autologem Serum des Patienten bei 37°C und 5% CO₂ inkubiert. Zweimal wöchentlich wird ein Mediumwechsel durchgeführt. Nach Erreichen des konfluenten Stadiums wird die Zellayer mit physiologischer Kochsalzlösung gewaschen und mittels Trypsin von der Zellkulturoberfläche geerntet. Nach einer weiteren Waschung werden 1 x 10⁵ Zellen in je ein Zellkulturgefäß überführt, das mit Agarose beschichtet ist. Nach einem Tag haben sich die ersten Zellen in Aggegaten angeordnet. Diese Aggregate werden regelmäßig mit frischem Medium versorgt und für mindestens 2 Wochen kultiviert.

Den Aufbau der erhaltenen Bandscheibenknorpelgewebe verdeutlicht die mikroskopische Aufnahme in Abb. 1, die den Querschnitt eines erfindungsgemäß hergestellten Bandscheibengewebes mit ECM als Zone verminderter Proliferation und Bildung von gewebespezifischen Matrixproteinen und P als äußere Proliferationszone zeigt.

In diesen in vitro Bandscheibengeweben wurde die Expression und Deposition von bandscheibenspezifischen Matrixbestandteile und regulatorische Proteine wie Aggrecan (Abb. 3a), hyalin-spezifische Proteoglykane (Abb. 3b), Kollagen Typ I (Abb. 3c), Kollagen Typ II (Abb. 3d) und Kollagen Typ III (Abb. 3e) nachgewiesen. Diese sind Bestandteile des nativen Bandscheibenknorpelgewebes *in vivo* und stellen die wichtigsten Strukturproteine dar, die für die Funktion des Bandscheibenknorpels von entscheidender Bedeutung sind.

Es war überraschend, dass die aus dem erkrankten Bandscheibengewebe isolierten Bandscheibenzellen, wenn diese als Gemisch aus Anulus Fibrosus und Nucleus Pulposus kultiviert werden, eine hohe Proliferationskapazität (Abb. 2) sowie ein sehr hohes Differenzierungspotential zur Bildung bandscheibenspezifischer Matrixproteine und regulatorischer Proteine aufweisen (siehe auch Abb. 3) und ihre Eigenschaften durch das Prozedere des Einfrierens und Auftauens erhalten werden können (siehe auch Abb. 5).

### Beispiel 4: Transplantation von Bandscheibenknorpelgewebe

Das in Beispiel 3 hergestellte Bandscheibenknorpelgewebe (ca. 10 bis 1000 Gewebestückchen aus je 1*10⁵ Zellen) vorzugsweise 100 Gewebestückchen wurde in physiologischer Kochsalzlösung bereits im Labor in eine Spritze aufgenommen und in den Zwischenwirbelraum von erkrankten oder geschädigten Bandscheiben mittels einer Punktionsnadel mit angeschrägtem Auslauf eingespritzt. Das erfindungsgemäß in vitro hergestellte Bandscheibenknorpelgewebe wird von den Patienten angenommen und gewährleistet neben der Erfüllung der mechanischen Funktion des hergestellten Gewebes die schnelle Integration der hergestellten Gewebestücke durch die proliferationsfähigen und migrationsfähigen Zellen in der äußeren Schicht der Aggregate sowie eine Regeneration des Bandscheibengewebes durch die Differenzierungsfähigkeit der enthaltenen Zellen. Somit erlaubt die Struktur und Funktion der Gewebestücke den schnellen Wiederaufbau von Bandscheibengewebe, eine schnelle Genesung der Patienten und die Wiederherstellung der Bandscheibenfunktion.

Abb. 4 zeigt fünf fusionierende Bandscheibengewebe. Es ist zu sehen, dass die Gewebekugeln aneinander haften und sozusagen verschmelzen, die Grenze zwischen zwei Bandscheibengeweben ist nicht mehr zu erkennen. Nach längerer Kultivierungszeit fusionieren die Bandscheibengewebe vollständig und es entsteht ein größeres in vitro Gewebestück. Der Aufbau der so erhaltenen.größeren Zellaggregate ist mit in vitro Bandscheibengewebe vergelichbar. Sie können bis zu maximal 99% ECM beinhalten und die enthaltenen Zellen sind vital.

## Patentansprüche

1. Verfahren zur Herstellung von autologen Gemisch-Bandscheibenzelltransplantaten, **dadurch gekennzeichnet, dass** aus degeneriertem, vorgefallenen Bandscheibengewebe und/oder erkranktem Bandscheibengewebe, vitale Bandscheibenzellen als Gemisch aus Bandscheibenteilen des Anulus Fibrosus und Nucleus Pulposus isoliert, nur unter Zusatz von körpereigenem Serum in einem Zellkulturmedium ohne Zusatz von exogenen wachstumsfördernden Verbindungen und ohne Zusatz von Antibiotika als Monolayer kultiviert und so Bandscheibenzelltransplantate erhalten werden, welche aus einer Bandscheibenzellsuspension bestehen.

2. Verfahren zur Herstellung von autologen Gemisch-Bandscheibenzelltransplantaten nach Anspruch 1, weiter **dadurch gekennzeichnet, dass** die besagten Bandscheibenzelltransplantate folgend zu einem autologen 3-dimensionalen Gewebe ohne die Verwendung von Trägermaterialien kultiviert werden.

3. Verfahren nach einem der Ansprüche 1 oder 2, weiter **dadurch gekennzeichnet, dass** die aus dem degenerierten, vorgefallenen Bandscheibengewebe isolierten Bandscheibenzellen während der Vermehrung in einem Zellkulturmedium, das 1-20% autologen Serumzusatz enthält und dessen Verhältnis von aplha-MEM-Medium und HAM-F12-Medium zwischen 2:1 und 1:2 beträgt, und bei 36.8-37°C, 5% Kohlendioxid Luftanteil und einer Luftfeuchte von 85-95% kultiviert werden.

4. Verfahren nach einem der Ansprüche 1 bis 3, **dadurch gekennzeichnet, dass** die isolierten Bandscheibenzellen nach ihrer Vermehrung in Monolayer in einem Zellkulturmedium, das 1-20% autologen Serumzusatz enthält und dessen Verhältnis von aplha-MEM-Medium und HAM-F12-Medium zwischen 2:1 und 1:2 beträgt, und bei 36.8-37°C, 5% Kohlendioxid Luftanteil und einer Luftfeuchte von 85-95% kultiviert und dadurch differenzierungsfähig sind und Matrixstrukturen bilden, die spezifische Bandscheibenmatrixproteine umfassen.

5. Verfahren nach den Ansprüchen 1 bis 4, **dadurch gekennzeichnet, dass** die isolierten Bandscheibenzellen nach deren Vermehrung in Monolayer in einer Lösung aus 10 % DMSO, 20 % Serum und 70 % Kulturmedium eingefroren und wieder aufgetaut werden, wobei deren Eigenschaften im Hinblick auf die Synthese von spezifischen Matrixkomponenten und Markern nicht verändert und Gewebestrukturen in vitro und in vivo aufgebaut werden, die aus Bandscheibenspezifischen Matrixproteinen bestehen.

6. Verfahren nach einem der Ansprüche 2 bis 5, **dadurch gekennzeichnet, dass** die aus dem Bandscheibengewebe isolierten Zellen in einem Kulturgefäß mit hydrophober Oberfläche und sich verjüngendem Boden kultiviert werden.

7. Autologes Gemisch-Bandscheibenzelltransplantat erhältlich nach einem der Ansprüche 1 bis 6.

8. Autologes Gemisch-Bandscheibenzelltransplantat nach Anspruch 7, **dadurch gekennzeichnet, dass** es in einer Spritze zur Transplantation vorliegt.

9. Verwendung des autologen Gemisch-Bandscheibenzelltransplantates nach Anspruch 7 zur in vitro Testung von Wirkstoffen.

10. Zelltherapeutische Zubereitungen umfassend autologe Gemisch-Bandscheibenzelltransplantate gemäß Anspruch 7.

11. Autologes Gemisch-Bandscheibenzelltransplantat gemäß einem der Ansprüche 7 bis 8 zur Verwendung in der Behandlung von Bandscheibendefekten.

## Claims

1. A method for the production of autologous mixed intervertebral disc cell transplants, **characterized in that** vital intervertebral disc cells are isolated from degenerated prolapsed intervertebral disc tissue and/or diseased intervertebral disc tissue as a mixture of parts from the intervertebral discs of the Annulus Fibrosus and the Nucleus Pulposus, cultured as a monolayer under addition of only endogenous serum in a cell culture medium without the addition of exogenous, growth-promoting compounds and without the addition of antibiotics, to obtain intervertebral disc cell transplants that consist of an intervertebral disc cell suspension.

2. The method for the production of intervertebral disc cell transplants of claim 1, further **characterized in that** said intervertebral disc cell transplants are subsequently cultured into an autologous three-dimensional tissue without the use of scaffolding materials.

3. The method of one of claims 1 or 2, further **characterized in that** during growth, the intervertebral disc cells, isolated from said degenerated prolapsed intervertebral disc tissue, are cultured in a cell culture medium that contains 1 - 20 % of autologous serum additive in which the ratio of alpha MEM medium and HAM F12 medium is between 2:1 and 1:2, at 36.8 - 37 °C, and in air containing 5 % carbon dioxide and with an air humidity of 85 - 95 %.

4. The method of one of claims 1 to 3, **characterized in that** the isolated intervertebral disc cells, following growth in a monolayer in a cell culture medium containing 1 - 20 % of autologous serum additive, where the ratio of alpha MEM and HAM F12 medium being between 2:1 and 1:2, at 36.8 - 37 °C and the air contains 5 % carbon dioxide and has air humidity of 85 - 95 %, are capable of differentiating and forming matrix structures comprising specific intervertebral disc matrix proteins.

5. The method of claims 1 to 4, **characterized in that** the isolated intervertebral disc cells following growth in a monolayer, are frozen and thereafter thawed in a solution of 10 % DMSO, 20 % serum and 70 % culture medium, whereby their properties with respect to the synthesis of specific matrix components and markers remain unchanged and tissue structures are formed in vitro and in vivo that consist of intervertebral disc-specific matrix proteins.

6. The method of one of claims 2 to 5, **characterized in that** the intervertebral disc cell transplants are cultured in a culture vessel with a hydrophobic surface and a tapering bottom.

7. An autologous mixed intervertebral disc cell transplant obtainable according to one of claims 1 to 6.

8. The autologous mixed intervertebral disc cell transplant of claim 7, **characterized in that** it is present in a syringe for transplantation.

9. Use of the autologous mixed intervertebral disc cell transplant of claim 7 for the in vitro testing of medical compounds.

10. A cell therapeutic formulation, comprising autologous mixed intervertebral disc cell transplants of claim 7.

11. The autologous mixed intervertebral disc cell transplant according to one of claims 7 to 8 for use in the treatment of intervertebral disc defects.

## Revendications

1. Procédé de fabrication de transplants de cellules discales mixtes autologues, **caractérisé en ce que** des cellules discales vitales sont isolées sous la forme d'un mélange des parties du disque vertébral Anulus Fibrosus et Nucleus Pulposus à partir d'un tissu discal dégénéré, en prolapsus et/ou d'un tissu discal malade, cultivées uniquement par ajout de sérum endogène dans un milieu de culture cellulaire sans ajout de composés exogènes favorisant la croissance et sans ajout d'antibiotiques sous la forme d'une monocouche, et des transplants de cellules discales sont ainsi obtenus, qui sont constitués par une suspension de cellules discales.

2. Procédé de fabrication de transplants de cellules discales mixtes autologues selon la revendication 1, en outre **caractérisé en ce que** lesdits transplants de cellules discales sont cultivés selon un tissu tridimensionnel autologue sans l'utilisation de matériaux supports.

3. Procédé selon l'une quelconque des revendications 1 ou 2, en outre **caractérisé en ce que** les cellules discales isolées à partir du tissu discal dégénéré, en prolapsus, sont cultivées pendant la multiplication dans un milieu de culture cellulaire qui contient 1 à 20 % d'ajout de sérum autologue et dont le rapport entre le milieu alpha-MEM et le milieu HAM-F12 est compris entre 2:1 et 1:2, et à 36,8 à 37 °C, une proportion de l'air de 5 % de dioxyde de carbone et une humidité de l'air de 85 à 95 %.

4. Procédé selon l'une quelconque des revendications 1 à 3, **caractérisé en ce que** les cellules discales isolées sont cultivées après leur multiplication en monocouche dans un milieu de culture cellulaire qui contient 1 à 20 % d'ajout de sérum autologue et dont le rapport entre le milieu alpha-MEM et le milieu HAM-F12 est compris entre 2:1 et 1:2, et à 36,8 à 37 °C, une proportion de l'air de 5 % de dioxyde de carbone et une humidité de l'air de 85 à 95 %, et sont ainsi capables de différenciation, et forment des structures matricielles qui comprennent des protéines matricielles discales spécifiques.

5. Procédé selon les revendications 1 à 4, **caractérisé en ce que** les cellules discales isolées sont congelées après leur multiplication en monocouche dans une solution de 10 % de DMSO, 20 % de sérum et 70 % de milieu de culture, puis décongelées, leurs propriétés au regard de la synthèse de composants matriciels spécifiques et de marqueurs n'étant pas modifiées et des structures tissulaires étant formées in vitro et in vivo, qui sont constituées de protéines matricielles discales spécifiques.

6. Procédé selon l'une quelconque des revendications 2 à 5, **caractérisé en ce que** les cellules isolées à partir du tissu discal sont cultivées dans un récipient de culture à surface hydrophobe et fond effilé.

7. Transplant de cellules discales mixtes autologues pouvant être obtenu selon l'une quelconque des revendications 1 à 6.

8. Transplant de cellules discales mixtes autologues selon la revendication 7, **caractérisé en ce qu'**il se présente dans une seringue pour la transplantation.

9. Utilisation du transplant de cellules discales mixtes autologues selon la revendication 7 pour l'analyse in vitro d'agents actifs.

10. Préparations thérapeutiques cellulaires comprenant des transplants de cellules discales mixtes autologues selon la revendication 7.

11. Transplant de cellules discales mixtes autologues selon l'une quelconque des revendications 7 à 8, destiné à une utilisation pour le traitement de défauts discaux.
